# EUROPÄISCHE PATENTSCHRIFT

(11) **EP 0 821 707 B1**
(45) Veröffentlichungstag und Bekanntmachung des Hinweises auf die Patenterteilung: **18.07.2001**
(21) Anmeldenummer: 96905726.4
(22) Anmeldetag: 14.03.1996
(51) Int. Cl.: C08G 65/20, C08G 65/10

(54) **VERFAHREN ZUR HERSTELLUNG VON POLYTETRAMETHYLENETHERGLYKOLDIESTER AN ALUMINIUM-MAGNESIUMSILIKAT KATALYSATOREN**
PROCESS FOR PRODUCING POLYTETRAMETHYLENE ETHER GLYCOL DIESTER ON ALUMINIUM MAGNESIUM SILICATE CATALYSTS
PROCEDE DE PRODUCTION DE DIESTER DE GLYCOL D'ETHER DE POLYTETRAMETHYLENE SUR DES CATALYSEURS EN SILICATE D'ALUMINIUM-MAGNESIUM

(30) Priorität: 16.04.1995 DE 19513493; 30.04.1995 DE 19515244
(43) Veröffentlichungstag der Anmeldung: 04.02.1998
(73) Patentinhaber: Korea PTG Co., Ltd., Ulsan, 680-150 (KR)
(72) Erfinder: Müller, Herbert, Dr., 67227 Frankenthal (DE)
(74) Vertreter: Weiss, Wolfgang, Dipl.-Chem. Dr.
(86) Internationale Anmeldenummer: DE9600452
(87) Internationale Veröffentlichungsnummer: WO9633232

(56) Entgegenhaltungen:
- DE-A- 2 916 653
- US-A- 4 127 513
- US-A- 5 210 283
- Kirk-Othmer Encyclopedia of Chemical Technology, 4.Ausgabe, Band 6, 1993, Seiten 381-398.

## Beschreibung

Die Erfindung betrifft ein Verfahren zur Herstellung von Polytetramethylenetherglykol(PTMEG)diester durch Polymerisation von Tetrahydrofuran (THF) in Gegenwart von Carbonsäureanhydrid mit einem neutralen oder schwach basischen Magnesium-Aluminium-Hydrosilikat-Katalysator, der durch Erhitzen auf 200-600°C aktiviert wurde. Die natürlich vorkommenden Mineralien Sepiolit und bevorzugt Attapulgit sind geeignete Ausgangsprodukte für die neuen erfindungsgemäßen Katalysatoren.

Die Polymersiation von THF durch Oxoniumionenkatalyse wurde als Ergebnis der grundlegenden Arbeiten von H. Meerwein et al. bekannt (Angew. Chemie 72, (1960), 927) und wird umfasend in der Monographie "Polytetrahydrofuran" von P. Dreyfuß, Gordon a. Breach Sc. Publishers, New York, London, Paris 1982 abgehandelt.

In der Deutschen Patentschrift PS 2916653 wird ein Polymerisationsverfahren für THF beschrieben, bei dem die Polymerisation von in einem separaten Schritt gereinigtem THF durch im Festbett angeordneter Bleicherde in Anwesenheit von Carbonsäureanhydrid erfolgt. Bleicherden sind natürlich vorkommende Aluminiumsilicate mit einer kryptokristallinen Dreischichtstruktur des Montmorillonit-Minerals. Das aus Lagerstätten gewonnene Mineral zeigt von Vorkommen abhängige variable physikalische und chemische Eigenschaften auf. Insbesondere die Aktivität der Katalysatoren ist nicht konstant, sondern schwankt von Charge zu Charge. Dies ist trotz des niedrigen Preises ein gravierender Nachteil für die gewerbliche Nutzung der Bleicherden als Katalysatoren. Ähnlich verhalten sich Kaolin und Zeolithe, die in der PCT-Anmeldung WO 94/05719 für die THF-Polymerisation vorgeschlagen wurden. Diese Mineralien entfalten katalytische Wirksamkeit erst nach einer Säurevorbehandlung. Nachteilig ist zusätzlich, daß nur extrem reines THF ein gewerblich nutzbares Polymerisat liefert. Der vorliegenden Erfindung lag nun die Aufgabe zugrunde die Polymerisation von THF in bezug auf die technische Ausführungsform zu vereinfachen und reproduzierbarer zu gestalten. Die Vorteile des in der Deutschen Patentschrift PS 2916653 beschriebenen Verfahrens insbeondere die der Festbettkatalyse, sollten aber erhalten bleiben.

Überraschenderweise zeigte sich, daß zu Formkörpern verpreßter im wesentlichen wasserfreier, neutraler-schwach basischer (pH=7-9,5) oder durch Säurebehandlung protonisierter Sepiolit und Attapulgit, die suspendiert oder besser in ein stationäres, fest angeordnetes Katalysatorbett eingebracht werden, eine Mischung aus THF und Carbonsäureanhydrid mit hoher Polymerisationsgeschwindigkeit in Polytetrabutylenetherglykolcarbonsäureester mit niedriger Farbzahl reproduzierbar und über ungewöhnlich lange Zeiträume umwandelt. THF bedarf anders als bei der Verwendung von Bleicherden, Zeolithen oder Kaolin keiner besonderen Reinigung. Es kann sogar mit wasserhaltigem THF (z.B. 1% Wasser) polymerisiert werden. Durch den Einsatz besonders von Attapulgit als Katalysator gelingt es Polymerisate herzustellen, die sich durch eine sehr niedrige Farbzahl, eine enge Molekulargewichtsverteilung und einem extrem niedrigen Gehalt an verunreinigenden Kronether auszeichnen. Attapulgit wird im erfindungsgemäßen Verfahren bevorzugt verwendet.

Der Katalysator weist eine nahezu unbegrenzte Lebensdauer auf und begründet u. a. auch dadurch die bessere Umweltverträglichkeit des neuen Verfahrens.

Sepiolit und Attapulgit sind in der Natur weit verbreitete hydratisierte Magnesium-Aluminiuim Hydrosilikate, die bereits ohne besondere Aktivierung durch Säuren die Tetrahydrofuran-Polymerisation bewirken. Im Vergleich zu den oben erwähnten Katalysatoren reagieren diese neuen Katalysatoren deshalb neutral oder schwach basisch. Sie enthalten keine Restsäure und sind dehalb nicht korrosiv. Für den großtechnischen Einsatz sind die deshalb auch aus ökonomischen Gründen zu empfehlen. In der USA-PS 5210283 wird auf die Wichtigkeit der Säurebehandlung bei Bleicherdekatalysatoren besonders hingewiesen und abgehandelt. Die bevorzugte Ausführungsform des erfindungsgemäßen Verfahrens mit neutralen oder schwach basischen Katalysatoren konnte erst nach Überwindung dieses Vorurteils entwickelt werden.
Katalysatoren für den erfindungsgemäßen Prozeß sind beispielsweise als Attapulgus Clay oder Folrida Erde bekannt. Die bevorzugten Katalysatoren bestehen überwiegend aus dem Mineral Attapulgit, das aus dreidimensionalen Ketten gebildet wird.

Im Gegensatz zu den Tonerden und Kaolin enthalten Attapulgit und Sepiolit Magnesium, ein stärker basisches Element als Aluminium, und sollte deshalb kationisch weniger wirksam sein. Überraschend erweisen sie sich aber bei der kationischen THF-Polymerisation als sehr aktive Katalysatoren.

Durch Extrusion und gezielte Calcination wird dem natürlichen Attapulgit Wasserentzogen. Durch Mahlen und Sieben erhält man Granulat oder pulverförmige Qualitäten, die im Festbett oder Suspensionsverfahren eingesetzt werden.

Die erfindungsgemäß zu verwendenden Katalysatoren sollen vor ihrer Verwendung einige Zeit z. B. 0,5 bis 10 Stunden bei Temperaturen zwischen 200 und 600°C getempert werden. Durch diese Maßnahmen wird der Wassergehalt erniedrigt und die Reaktivität formiert.

Das im Katalysatorfestbett eingesetzte Granulat hat z.B. ein Komgrößenspektrum von 4/8 mesh = 2,4 - 4,75 mm. Man kann auch Attapulgitpulver mit Wasser anteigen, zu Formkörpem verpressen und calcinieren.

Für die Polymerisation des THF wird nur eine geringe Menge des Kastalysators benötigt Die trockenen Katalysatorkörper werden in einem Reaktionsgefäß, beispielsweise einem Röhren- oder Schachtofen aufgeschüttet Die gewählte Dimension der Schüttung wird vorzugsweise von der Notwendigkeit bestimmt, die Polymerisationswärme abzuführen. Es kann auch nützlich sein, das Reaktionsprodukt ganz oder zum Teil im Kreis über die Schüttung zu pumpen, um in einem Wärmeaustauscher durch Kühlen oder Erwärmen für eine isotherme Reaktionsführung längs des Ofens zu sorgen. Im allgemeinen genügt ein Kreisstrom der etwa das 3 bis 10 fache des Reaktorvolumens stündlich ausmacht Bei kontinuierlicher Polymerisation fügt man dem Kreislaufprodukt das 0,01 - 0,1 fache der stündlichen Kreislaufmenge Frischzulauf als THF und Carbonsäureanhydrid zu.

Ein geeigneter Reaktor für die erfindungsgemäße Polymerisation ist auch ein rotierender Korb gefüllt mit Granulat, der sich in einem thermostatisierbaren Reaktor befindet, der zusätzlich noch mit einem Rührpaddel ausgestattet sein kann.

Unerwartet und technisch fortschrittlich im Vergleich zu der bisher bekannten Verfahrensweise wie sie z.B. indem Deutschen Patent2916653 beschrieben wird, entstehen nach dem erfindungsgemäßen Verfahren Produkte mit einer sehr engen Molekulargewichtsverteilung und einem vernachlässigbar geringen Anteil von weniger als 0,1 Gew.% an verunreinigenden Kronether. Während marktgängiges Polytetramethylenetherglykol (PTMEG) mit der Molmasse 1000 durch den Einheitlichkeitsquotienten M_{w}/Mₙ von 1,6 bis 1,8 charakterisiert wird, entsteht erfindungsgemäß ein Produkt mit dem Quotienten M_{w}/Mₙ von 1,2 bis 1,4. Dieses eignet sich besonders fnr die Herstellung elastischer Polyurethanfaser oder thermoplastischer Polyurethane mit gutem Tieftemperaturverhalten.

Der im wesentlichen wasserfreie Attapulgit und Sepiolit entfaltet erst in Gegenwart des Promotors Carbonsäureanhydrid seine katalytische Wirkung. Vorteilhafterweise verwendet man solche Carbonsäureanhydride, die sich von aliphatischen oder aromatischen Poly- und/oder vorzugsweise Monocarbonsäuren mit 2 bis 12, vorzugsweise 2 bis 8 Kohlenstoffatomen ableiten. Genannt seien z. B. Essigsäureanhydrid, Propionsäureanhydrid, Buttersäureanhydrid oder Acrylsäure- und Methacylsäureanhydrid sowie Bemsteinsäureanhydrid. Auch gemischte Anhydride und Anhydridgemische sind verwendbar. Bevorzugt wird schon aus Preisgründen Essigsäureanhydrid.

Wie bereits erwähnt, können nach dem erfindungsgemäßen Verfahren Diester von Polybutylenglykolether von beliebigem Polymerisationsgrad hergestellt werden. Die Carbonsäureanhydridkonzentration der Polymerisationsmischung bestimmt den Polymerisationsgrad. Je niedriger die Anhydridkonzentration gewählt wird, um so höhere Molekulargewichte werden erreicht und umgekehrt. Als Orientierungswerte für eine Reaktionstemperatur von 50°C können folgende Angaben dienen:

| | | | | |
|---|---|---|---|---|
| Polymerisationsgrad | 8 | 9 | 10 | 24 |
| | | | | |
| Gew.% Essigsäureanhydrid im Polymerisationsansatz | 10 | 8,5 | 6,8 | 3 |

Zur Durchführung der Polymerisation werden Festbettkatalysatoren in einem geeigneten Reaktionsgefäß z.B. bei der bevorzugten Ausführungsform der Polymerisation in Abwesenheit einer Gasphase nach der sogenannten Sumpffahrweise mit dem Reaktionsgemisch z.B. durch Umpumpen in Berührung gebracht. Die bei der Polymerisation entstehende Reaktionswärme wird in geeigneter Weise abgeführt. Man kannn auch bei der einfachsten Form der Ausführung des erfindungsgemäßen Verfahrens die Katalysatorschüttung einfach mit dem Polymerisationsgemisch überstellen und die Umsetzung z.B. adiabatisch durchführen. Bei sehr aktiven Katalysatoren begrenzt siedendes THF die Maximaltemperatur bei ca. 65°C. Bei dieser Reaktionsführung ist die Polymerisation nach ca. 30 bis 60 Minuten zu Ende.

Im Allgmeinen wird man die Polymerisation bei Drücken zwischen 0 bis 25 bar und bei Temperaturen zwischen 10 bis 60°C ausüben. Niedrigere oder höhere Drücke und Temperaturen bringen keine Vorteile.

In den meisten Fällen wird man die Polymerisation bis zum vollständigen Umsatz des Carbonsäureanhydrids ablaufen lassen. Je nach Polymerisationstemperatur sind dann, wenn im Bereich von 30 bis 55°C polymerisiert wurde, 40 bis 75 Gew.% des eingesetzten THF umgesetzt. Nicht reagiertes THF gewinnt man bei der destillativen Aufarbeitung des Reaktionsproduktes wieder, und kann es erneut ohne Nachteile für spätere Polymerisationen verwenden.

Die nach dem erfindungsgemäßen Verfahren erhaltenen Polybutylenetherglykoldiester lassen sich nach den bekannten Mehoden verseifen, oder auch z.B. nach der Methode des US-PS 2499725 mit beispielsweise Methanol umestem. Besonders für die großtechnische Ausübung des Verfahrens empfiehlt sich die hydrierende Umesterung, wie sie in der US PS 4608422 beschrieben ist. Weitere Methoden sind in den Patentschriften DE 2760272, EP 0185553 und EP 0038009 beschrieben. Am einfachsten überführt man die Diester nach der von Adkins eingeführten Methode der Esterhydrierung mit Kupfer-Chromoxid-Katalysatoren in die Diolform. Geeignete Katalysatoren werden im Handel angeboten. So z.B. die 5-15% Barium enthaltenden Kupferchromitkatalysatoren, die bei 220°C und 250 bar Wasserstoffdruck ohne Lösungsmittel oder auch in Gegenwart von Methanol oder Äthanol die Diester verlustlos in das PTMEG überführen. Dieses ist dann z.B. mit Molekulargewichten 800-3000 geeignet für die Herstellung von Polyestem oder Polyurethanen. Die nach dem erfindungsgemäßen Verfahren erhaltenen Produkte können nach der Adkinsmethode zu Produkten mit ausreichend niedriger Restesterzahl von < 1 mg KOH/g hydriert werden. PTMEG-diacetate, die durch andere Polymerisationsmethoden (z.B. durch Montmorillonitkatalyse) entstehen, ergeben Polytetramethylenetherglykole mit Restesterzahlen von über 1 mg KOH/g.

Die folgenden Beispeile sollen das erfindungsgemäße Verfahren näher erläutern ohne es zu begrenzen. Die genannten Teile sind Gewichtsteile: sie verhalten sich zu Volumenteilen wie Kilogramm zu Litern.

### Beispiel 1

Attapulgus Clay/Floridin 4/8 mesh LVM (Granulat) der Firma Chemie-Mineralien AG u. Co KG, Bremen wurde2 Stunden lang bei 300°C calciniert und im Exiccatorabgekühlt. 150cm³ des wasserfreien Granulats werden in eine Duranglas-Laborflasche der Firma Schott, Mainz, gefüllt, im Wasserbad auf 50°C vorgewärmt und mit 300 g einer Mischung, die aus 91,5 Gew.% techn. THF und 8,5 Gew.% Essigsäureanhydrid besteht, überstellt. Die dicht mit einer Polypropylenschraubkappe verschlossene Flasche wird im Wasserbad bei 50°C 4 Stunden lang langsam um die Längsachse gedreht, um die Katalysatorschüttung in leichter Bewegung zu halten.

Das Reaktionsgemisch wurde danach vom Katalysator dekantiert und untersucht Aus der Säurezahl des Reaktionsproduktes läßt sich ein etwa 99%iger Umsatz des Essigsäureanhydrids ableiten.

Bei 150°C und 1 mbar dampft man das unumgesezte THF im Filmverdampfer vom entstandenen Polytetramethylenetherglykoldiacetat ab, das zu 56 Gew.% in der Reaktionslösung enthalten war. Die Verseifungszahl des Diesters beträgt 152,6 mg KOH/ g was einem Molekulargewicht von 734 g/mol entspricht. Durch Umestern mit der gleichen Menge Methanol in Gegenwart von z.B. 0,01 Gew.% Natriummethylat erhält man daraus PTMEG mit der Hydroxylzahl 173 mg KOH/g und der Farbzahl 5 APHA. Das Produkt zeigt eine sehr enge Molekulargewichtsverteilung. Der Heterogenitätsquotient (Polydisperistät) M_{w}/Mₙ beträgt nur 1,20. Der Gehalt an oligomeren cyclischen Ethem ist kleiner 0,01 Gew.%.

### Beispiel 2

Die Polymerisation wird in der im Beispiel beschriebenen Versuchsanordnung bei 50°C mit einem käuflichen Granulat Attapulgus Clay 4/8 mesh LVM, Chemie Mineralien AG, Bremen durchgeführt. Der Katalysator wird bei 250°C 3 Stunden bis zur Gewichtskonstanz getrocknet. Für die Polymerisation wird eine 3 Gew.%ige Essigsäureanhydridlösung in techn. THF verwendet. Nach 2,5 Stunden Polymerisationszeit hat Essigsäureanhydrid zu über 99% reagiert, und die Polymerenlösung enthält 53 Gew.% PTMEG-Diacetat mit der Esterzahl 61,2 (entspricht dem Molekulargewicht 1824 g/mol). Durch Hydrierung am Festbett in der Sumpffahrweise wird dieses in 60 Gew.%iger Methanollösung mit durch 11 Gew.% Bariumoxid aktivierten Kupferchromit-Katalysator bei 220°C und 250 bar Wasserstoff in PTMEG mit der Hydroxylzahl 64,1 (Molekulargewicht 1750g/mol) überführt. Das durch Eindampfen (1 mbar, 190 °C) gewonene Polymere ist sehr einheitlich und zeigt in der GPLC-Analyse die Polydisperistät M_{w}/Mₙ = 1,5. Die Farbzahl liegt bei 5 APHA.

### Beispiel 3

Wie im Beispiel 1 beschrieben, wird THF mit anderen Säureanhydriden polymerisiert. Verwendet man an Stelle von 8,5 Gew.% Essigsäureanhydrid im Reaktionsansatz:
1. 16 Gew.% Buttersäureanhydrid,
2. 24 Gew.% 2-Ethylhexansäureanhydrid oder
3. 21 Gew.% Benzoesäureanhydrid,
   so erhält man Polymerlösungen von

1. 55 Gew.% PTMEG-dibuttersäureester,
2. 60 Gew.% PTMEG-di-2-ethylhexansäureester und
3. 58 Gew.% PTMEG-Dibenzoat.

Die Ester lassen sich wie oben beschrieben nach verschiedenen Methoden in PTMEG mit den Hydroxylzahlen
1. 168 mg KOH/g
2. 170 mg KOH/g und
3. 166 mg KOH/g
   überführen.

### Beispiel 4

Attapulgus Clay/Floridin 4/8 mesh LVM (Granulat) der Firma Chemie-Mineralien AG u. Co KG, Bremen wurden mit einer 0,2 Gew.%igen Salzsäure überstellt und die überschüssige Lösung am Büchnertrichter abgesaugt und dann mit destilliertem Wasser gewaschen. Das Granulat wurde danach 2 Stunden lang bei 300°C calciniert und im Exiccator abgekühlt. 150 cm³ des wasserfreien Granulats werden in eine Duranglas-Laborflasche der Firma Schott, Mainz, gefüllt, im Wasserbad auf 50°C vorgewärmt und mit 300 g einer Mischung, die aus 91,5 Gew.% techn. THF und 8,5 Gew.% Essigsäureanhydrid besteht, überstellt. Die dicht mit einer Polypropylenschraubkappe verschlossene Flasche wird im Wasserbad bei 50°C 4 Stunden lang langsam um die Längsachse gedreht, um die Katalysatorschüttung in leichter Bewegung zu halten.

Das Reaktionsgemisch wurde danach vom Katalysator dekantiert und untersucht. Aus der Säurezahl des Reaktionsproduktes läßt sich ein etwa 99%iger Umsatz des Essigsäureanhydrids ableiten.

Bei 150°C und 2 mbar dampft man das unumgestezte THF vom entstandenem Polytetrarnethylenetherglykoldiacetat ab, das zu 56 Gew.% in der Reaktionslösung enthalten war. Die Verseifungszahl des Diesters beträgt 154 mg KOH/g was einem Molekulargewicht von 730 g/mol entspricht. Durch Umestern mit der gleichen Menge Methanol in Gegenwart von z.B. 0,01 Gew.% Natriummethylat erhält man daraus PTMEG mit der Hydroxylzahl 173 mg KOH/g und der Farbzahl 5 APHA. Das Produkt zeigt eine sehr enge Molekulargewichtsverteilung. Der Heterogenitätsquotient (Polydisperistät) M_{w}/Mₙ beträgt nur 1,20. Der Gehalt an oligomeren cyclischen Ethem ist kleiner 0,01 Gew.%.

## Patentansprüche

1. Verfahren zur Herstellung von Polytetramethylenetherglykoldiester mit der Formel R-CO-O(-CH₂-CH₂-CH₂-CH₂-O)ₙ-COR₁, in der R und R₁ identisch oder verschieden sind und einen Alkylrest oder ein Derivat davon bedeuten, und n eine ganze Zahl von 2 bis 200 ist, durch Polymerisation von Tetrahydrofuran in Gegenwart eines Polymerisationskatalysators und eines Carbonsäureanhydrids,
**dadurch gekennzeichnet,**
dass als Katalysator ein neutrales, schwach basisches oder protoniertes calciniertes Magnesium-Aluminium-Hydrosilicat, welches ein Attapulgit oder Sepiolit ist, verwendet wird.

2. Verfahren nach Anspruch 1,
**dadurch gekennzeichnet,**
dass der Katalysator weniger als 2 % Wasser enthält.

3. Verfahren nach Anspruch 1 bis 2,
**dadurch gekennzeichnet,**
dass der Katalysator im Festbett angeordnet ist und die Mischung aus Tetrahydrofuran und dem Carbonsäureanhydrid über dieses Festbett geleitet wird.

4. Verfahren nach Anspruch 1 bis 3,
**dadurch gekennzeichnet,**
dass der Katalysator vor der Verwendung 0,1 bis 5 Stunden einer thermischen Vorbehandlung bei 200 bis 600 °C unterworfen wurde.

## Claims

1. A process for the preparation of polytetramethylene ether glycol diesters of the formula
R-CO-O(-CH₂-CH₂-CH₂-CH₂-O)ₙ-COR₁,
in which R and R₁ are identical or different and are an alkyl radical or a derivative thereof, and n is an integer from 2 to 200, by polymerisation of tetrahydrofuran in the presence of a polymerisation catalyst and a carboxylic acid anhydride,
**characterised in that**
the catalyst is a neutral, weakly basic or protonated, calcined magnesium-aluminium hydrosilicate which is an attapulgite or sepiolite.

2. The process of claim 1,
**characterised in that**
the catalyst has a water content of less than 2 %.

3. The process according to claims 1 to 2,
**characterised in that**
the natural minerals attapulgite and sepiolite are used.

4. The process according to claims 1 to 4,
**characterised in that**
the catalyst is subjected to thermal pre-treatment at 200 to 600 °C for 0.1 to 5 hours before it is used.

## Revendications

1. Procédé pour la préparation de diester de polytétraméthylène-étherglycol répondant à la formule R-CO-O(-CH₂-CH₂-CH₂-CH₂-O)ₙ-COR₁, dans laquelle R et R₁ sont identiques ou différents et représentent un radical alkyle ou un de ses dérivés, et n représente un nombre entier de 2 à 200, par polymérisation de tétrahydrofuranne en présence d'un catalyseur de polymérisation et d'un anhydride d'acide carboxylique, caractérisé en ce qu'on utilise, à titre de catalyseur, un hydrosilicate de magnésium - aluminium calciné neutre, faiblement basique ou protoné, à savoir une attapulgite ou une sépiolite.

2. Procédé selon la revendication 1, caractérisé en ce que le catalyseur contient de l'eau en une quantité inférieure à 2 %.

3. Procédé selon les revendications 1 à 2, caractérisé en ce que le catalyseur est disposé sur un lit fixe et le mélange de tétrahydrofuranne et de l'anhydride d'acide carboxylique est guidé pardessus ce lit fixe.

4. Procédé selon les revendications 1 à 3, caractérisé en ce qu'on a soumis le catalyseur avant son utilisation, pendant un laps de temps de 0,1 à 5 heures, à un traitement thermique préalable à une température de 200 à 600°C.
